# EUROPEAN PATENT APPLICATION

(11) **EP 2 133 035 A1**
(43) Date of publication of application: **16.12.2009**
(21) Application number: 08703741.2
(22) Date of filing: 23.01.2008
(51) Int. Cl.: A61B 18/14, A61B 1/00

(54) **TREATMENT DEVICE**

(30) Priority: 30.03.2007 JP 2007094127
(71) Applicant: Olympus Medical Systems Corp., Tokyo 151-0072 (JP)
(72) Inventor: NAITO, Kimihiko, Tokyo 192-8512 (JP)
(74) Representative: von Hellfeld, Axel
(86) International application number: PCT/JP2008/050911
(87) International publication number: WO 2008/126434

(57) **Abstract**

The present invention is aimed at providing an accessory wherein a treatment member can be housed within a bending portion without preventing a bending actuation of a bending portion. An accessory includes bending parts 68a, 68b, 68c, 68d, 68e forming a bending portion wherein each bending part includes a cylindrical portion and a coupling end portion, the cylindrical portions are arranged side by side in an axial direction, and both the coupling end portions adjacent to each other are coupled to each other so as to be rotatable relative to each other, a cylindrical guide member 78, 82, 84 extending within the bending portion, a treatment member 86, 88 configured to be inserted through the guide member 78, 82, 84 so as to be movable forward and backward and placed in a housing position where the whole treatment member 86, 88 is housed within at least a given bending part 68a of the bending parts 68a, 68b, 68c, 68d, 68e out of the given bending part 68a and the coupling end portion provided in the bending part adjacent to the given bending part and arranged on a side close to the given bending part, and a stopper portion 92 provided on the guide member 78, 82, 84 and configured to be brought into contact with the treatment member 86, 88 to position the treatment member 86, 88 on the housing position.

## Description

### Technical Field

The present invention relates to an accessory wherein a bending portion is actuated to be bent to move the treatment member to treat a living tissue with the treatment member.

### Background Art

Various accessories are used, wherein a bending portion is actuated to be bent to move a treatment member to treat a living tissue with the treatment member.

As such an accessory, a high-frequency incision accessory for an endoscope is disclosed in Jpn. Pat. Appln. KOKAI Publication No. 2005-261521. In the high-frequency incision accessory, a bending portion in which bending parts are coupled to each other so as to be rotatable relative to each other is provided on the distal end portion of a long and flexible insertion tube portion. A rod-shaped high-frequency incision electrode is protruded toward the distal end side from a distal end tip of the bending part arranged on the most distal end. The high-frequency incision accessory is inserted through an accessory channel of an endoscope and projected from the distal end portion of the endoscope. After that, while a high-frequency current is supplied to the high-frequency incision electrode, the bending portion is actuated to be bent to move the high-frequency incision electrode to incise a living tissue with the high-frequency incision electrode.

In a magnetic resonance observation apparatus disclosed in Jpn. Pat. Appln. KOKAI Publication No. 9-103415, in an insertion portion configured to be inserted into a cavity in the body, a bending tube portion in which bending parts are coupled to each other so as to be rotatable relative to each other is coupled to the distal end portion of a long and flexible insertion tube portion. A RF signal receiving antenna system is provided along the central axis within a circularly cylindrical distal end bending part arranged on the most distal end, and the whole RF signal receiving antenna system is housed within the distal end bending part.

### Disclosure of Invention

In the high-frequency incision accessory disclosed in Jpn. Pat. Appln. KOKAI Publication No. 2005-261521, the high-frequency incision electrode is fixed to the distal end tip and not housed within the bending potion differing from the RF signal receiving antenna system of the magnetic resonance observation apparatus disclosed in Jpn. Pat. Appln. KOKAI Publication No. 9-103415.

The present invention is aimed at providing an accessory wherein a treatment member can be housed within a bending portion without preventing a bending actuation of a bending portion.

In an aspect of the present invention, an accessory includes: bending parts forming a bending portion wherein each bending part includes a cylindrical portion and a coupling end portion, the cylindrical portions are arranged side by side in an axial direction, and both the coupling end portions adjacent to each other are coupled to each other so as to be rotatable relative to each other; a cylindrical guide member extending within the bending portion; a treatment member configured to be inserted through the guide member so as to be movable forward and backward and placed in a housing position where the whole treatment member is housed within at least a given bending part of the bending parts out of the given bending part and the coupling end portion provided in the bending part adjacent to the given bending part and arranged on a side close to the given bending part; and a stopper portion provided on the guide member and configured to be brought into contact with the treatment member to position the treatment member on the housing position.

In the aspect, when the treatment member is positioned on the housing position by the stopper portion, the whole treatment member is housed within the given bending part out of the given bending part and the coupling end portion provided in the bending part adjacent to the given bending part and arranged on the side close to the bending part, and not placed within the cylindrical portion of the bending part adjacent to the given bending part. Therefore, it is prevented that a bending actuation of the bending portion is obstructed due to interference between the treatment member and the bending part.

In a preferred aspect of the present invention, the whole treatment member is configured to be housed within the given bending part when the treatment member is placed on the housing position.

In the preferred aspect, the whole treatment member is housed within the given bending part on the housing position, and therefore, it is securely prevented that a bending actuation of the bending portion is obstructed due to interference between the treatment member and the bending part.

In a preferred aspect of the present invention, the treatment member is configured to be housed within the given bending part other than the bending part arranged on a distal end out of the bending parts.

In the preferred aspect, the treatment member is configured to be housed within the backward bending part. Therefore, it is possible to make the forward bending part smaller than the backward bending part, and make a turning radius of the bending portion shorter.

In a preferred aspect of the present invention, a part of the guide member, in which the treatment member is adapted to be moved forward and backward, is hard.

In the preferred aspect, the part of the guide member, in which the treatment member is adapted to be moved forward and backward, is hard. Therefore, it is prevented that the guide member is damaged.

In a preferred aspect of the present invention, the accessory is configured to be inserted through an accessory channel of an endoscope and projected from a distal end portion of the endoscope, and the distal end portion of the accessory has a shape configured not to block an optical axis passing through a focal point of an observation optical system of the endoscope and a distal end of the accessory when the accessory is projected at a maximum from the distal end portion of the endoscope.

In the preferred aspect, the distal end of the accessory can be visually recognized even when the accessory is projected at a maximum from the distal end portion of the endoscope. Therefore, it is possible to secure sufficient visibility and securely perform treatment.

### Brief Description of Drawings

FIG. 1 is a perspective view showing an endoscope treatment system according to a first embodiment of the present invention;
FIG. 2 is a perspective view showing a distal end side portion of an active high-frequency accessory according to the first embodiment of the present invention;
FIG. 3 is a longitudinal cross-sectional view showing the distal end side portion of the active high-frequency accessory in a state where a high-frequency electrode is projected according to the first embodiment of the present invention;
FIG. 4 is a longitudinal cross-sectional view showing the distal end side portion of the active high-frequency accessory in a state where the high-frequency electrode is housed according to the first embodiment of the present invention;
FIG. 5 is a longitudinal cross-sectional view showing the distal end side portion of the active high-frequency accessory in a state where a bending portion is actuated to be bent according to the first embodiment of the present invention;
FIG. 6 is a side view showing a proximal end side portion of the active high-frequency accessory according to the first embodiment of the present invention;
FIG. 7 is a back view showing the proximal end side portion of the active high-frequency accessory according to the first embodiment of the present invention;
FIG. 8 is a schematic view showing a shape of a distal end portion of the active high-frequency accessory according to the first embodiment of the present invention;
FIG. 9 is a longitudinal cross-sectional view showing a distal end portion of an active high-frequency accessory in a state where a bending portion is actuated to be bent according to a modified example of the first embodiment of the present invention;
FIG. 10 is a longitudinal cross-sectional view showing a stopper portion and a surrounding portion thereof of an active high-frequency accessory according to a second embodiment of the present invention;
FIG. 11 is a longitudinal cross-sectional view showing a distal end side portion of an active high-frequency accessory according to a third embodiment of the present invention;
FIG. 12 is a longitudinal cross-sectional view showing a distal end side portion of an active high-frequency accessory according to a fourth embodiment of the present invention;
FIG. 13 is a longitudinal cross-sectional view showing a distal end side portion of an active high-frequency accessory according to a fifth embodiment of the present invention;
FIG. 14 is a schematic view showing a shape of a distal end portion of an active high-frequency accessory according to a sixth embodiment of the present invention;
FIG. 15 is a schematic view showing a shape of a distal end portion of an active high-frequency accessory according to a seventh embodiment of the present invention.

### Best Mode for Carrying Out the Invention

Hereinafter, each of embodiments of the present invention will be explained referring to the drawings.

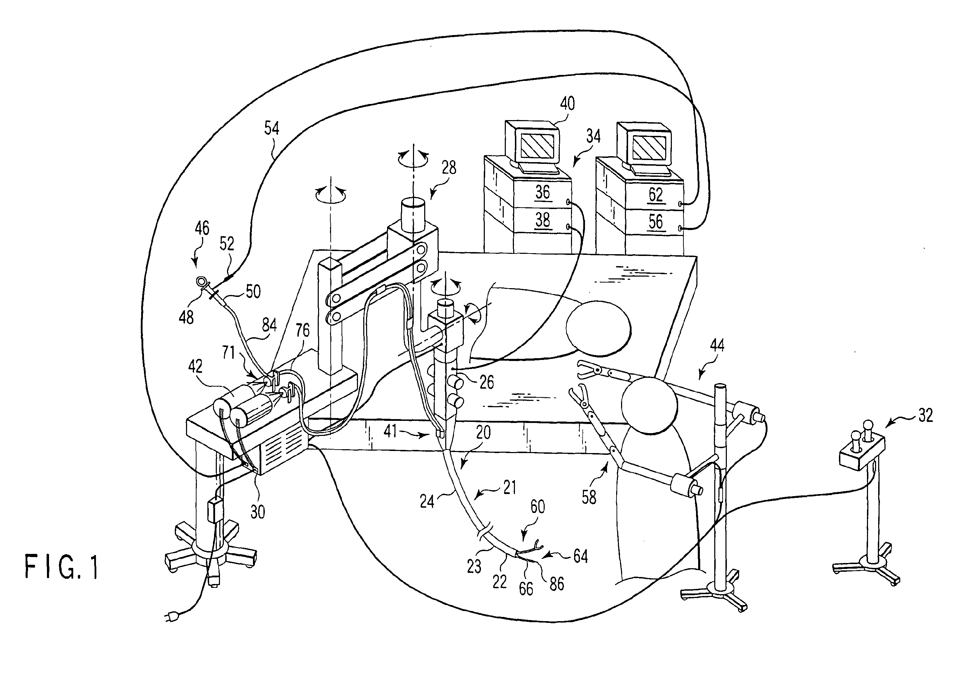

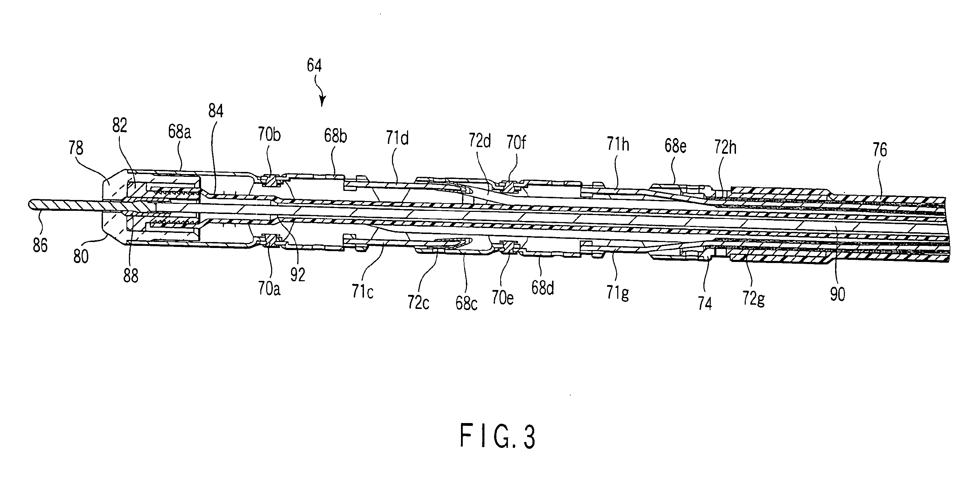

FIGS. 1 to 8 show a first embodiment of the present invention.

Referring to FIG. 1, a schematic structure of an endoscope treatment system according to the embodiment will be explained.

An active endoscope 20 of the endoscope treatment system includes an endoscope insertion portion 21 configured to be inserted into a cavity in the body. In the endoscope insertion portion 21, a rigid distal end rigid portion 22, an endoscope bending portion 23 configured to be actuated to be bent, and a long and flexible endoscope insertion tube portion 24 are provided in order from the distal end side, and an endoscope operation portion 26 is coupled to the proximal end portion of the endoscope insertion portion 21. The endoscope operation portion 26 is configured to be attached to and detached from the distal end portion of a movable type of endoscope stand 28 and is attached thereto, and the movable type of endoscope stand 28 is configured to move and fix the endoscope operation portion 26 to and in any position.

A group of endoscope angle wires for operating the endoscope bending portion 23 to be bent is inserted through the endoscope bending portion 23 and the endoscope insertion tube portion 24. The group of endoscope angle wires is put into the endoscope operation portion 26 from the endoscope insertion tube portion 24, and coupled to an endoscope wire actuating apparatus within the endoscope operation portion 26. The endoscope wire actuating apparatus is connected to an endoscope control apparatus of a control unit 30 provided on the movable type of endoscope stand 28, and a joystick 32 is connected to the endoscope control apparatus. When the joystick 32 is operated, the endoscope wire actuating apparatus moves the endoscope angle wires forward and backward, and the endoscope bending portion 23 of the active endoscope 20 is actuated to be bent.

Moreover, the active endoscope 20 is connected to a light source apparatus 36 and a display processor 38 carried on an endoscope trolley 34. Illumination light is supplied from the light source apparatus 36 to the active endoscope 20, and emitted from the distal end portion of the active endoscope 20. An image signal of an observation image obtained by an image pick-up unit of an observation optical system in the distal end portion of the active endoscope 20 is output to the display processor 38, and the observation image is displayed on a display apparatus 40.

Furthermore, an accessory insertion inlet 41 is provided on the endoscope operation portion 26 of the active endoscope 20, and an accessory channel is extended from the accessory insertion inlet 41 to the distal end portion of the active endoscope 20.

An active high-frequency accessory 64 is adapted to be inserted through the accessory channel of the active endoscope 20. In the active high-frequency accessory 64, a high-frequency electrode 86, a bending portion 66 configured to be actuated to be bent, and a long and flexible insertion tube portion 76 are provided in order from the distal end side.

A group of angle wires 71 for actuating the bending portion 66 to be bent is inserted through the bending portion 66 and the insertion tube portion 76. The insertion tube portion 76 is put out from the accessory insertion inlet 41 and extended to a wire actuating apparatus 42 provided on the movable type of endoscope stand 28, and the group of angle wires 71 is put out from the proximal end portion of the insertion tube portion 76 and coupled to the wire actuating apparatus 42. The wire actuating apparatus 42 is connected to a control apparatus of the control unit 30, and a master arm 44 is connected to the control apparatus. The master arm 44 has a similar degree of freedom to that of the bending portion 66 of the active high-frequency accessory 64. When the master arm 44 is operated, the wire actuating apparatus 42 moves the group of angle wires 71 forward and backward, and the bending portion 66 is actuated to be bent following operation to the master arm 44.

In addition, an operation wire for operating the high-frequency electrode 86 to be moved forward and backward and supplying a high-frequency current to the high-frequency electrode 86 is inserted through the bending portion 66 and the insertion tube portion 76. The operation wire is inserted into a resin tube 84, and the resin tube 84 is extended from the proximal end portion of the insertion tube portion 76. An operation portion 46 is coupled to the proximal end portion of the resin tube 84, and the proximal end portion of the operation wire is coupled to a slider 48 in the operation portion 46. When the slider 48 is moved forward and backward relative to an operation portion main portion 50, the operation wire is moved forward and backward. Moreover, a connecting terminal 52 electrically connected to the operation wire is provided on the slider 48. The connecting terminal 52 is connected to a high-frequency power source 56 through a power cord 54, and a high-frequency current is output from the high-frequency power source 56 through the power cord 54 and the operation wire to the high-frequency electrode 86. It is noted that a footswitch is connected to a power source control apparatus of the high-frequency power source 56. When the footswitch is operated, the high-frequency power source 56 is actuated and stopped.

It is noted that an active grasping forceps is configured to be inserted through a forceps channel of the active endoscope 20 and operated by a forceps master arm 58.

Moreover, a personal computer is connected to the control unit 30 and configured to perform various kinds of arithmetic and storage processing.

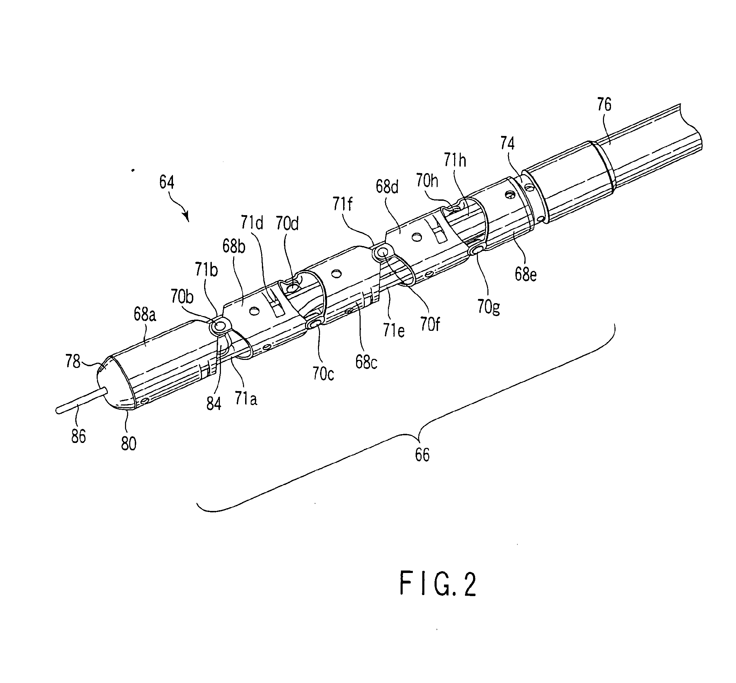

Referring to FIGS. 2 to 5, a structure of the distal end side portion of the active high-frequency accessory 64 will be explained in detail.

In the bending portion 66 of the active high-frequency accessory 64, a first to a fifth substantially circularly cylindrical bending part 68a, 68b, 68c, 68d, 68e made of metal is coupled to each other so as to be rotatable relative to each other in order from the distal end side. That is, a pair of tongue portions as a coupling end portion is protruded symmetrically with each other about the central axis from the proximal end surface of a circularly cylindrical portion as a cylindrical portion of the first bending part 68a and the distal end surface of a circularly cylindrical portion of the second bending part 68b, respectively, and the pairs of tongue portions of both the bending parts 68a and 68b are overlapped with each other and coupled to each other so as to be rotatable relative to each other by rivets made of metal. In this way, a first pair of coupling portions 70a and 70b is formed. The second to the fifth bending part 68b ... 68e is also coupled to each other through a second to a fourth pair of coupling portions 70c and 70d, 70e and 70f, 70g and 70h in the similar manner. The first to the fourth pair of coupling portions 70a ... 70f is arranged so as to be shifted by substantially 180 degree in the peripheral direction in order, and rotational directions of the bending parts 68a ... 68e arranged on the distal end side and the proximal end side of the given bending part 68a ... 68e are substantially orthogonal to each other. The proximal end portion of the fifth bending part 68e arranged on the most proximal end is connected to the distal end portion of the insertion tube portion 76 through a connecting tube 74.

The distal end portions of the first pair of angle wires 71a and 71b made of metal are fixed to the inner peripheral surface of the first bending part 68a on the positions which are shifted by substantially 90 degree in the peripheral direction from the positions of the first pair of coupling portions 70a and 70b arranged on the proximal end side thereof, respectively. A first pair of flexible coil sheaths 72a and 72b made of metal is provided onto the outside of the first pair of angle wires 71a and 71b, respectively, and the distal end portions of the first pair of coil sheaths 72a and 72b are fixed to the inner peripheral surface of the second bending part 68b. As is similar to this, the distal end portions of a second to a fourth pair of angle wires 71c and 71d, 71e and 71f, 71g and 71h are fixed to the second to the fourth bending part 68b ... 68d, and the second to the fourth pair of coil sheaths 72c and 72d, 72e and 72f, 72g and 72h is provided onto the outside of the second to the fourth pair of angle wires 71c ... 71h. Regarding the first to the fourth pair of angle wires 71a ... 71h, when one angle wire 71a ... 71h is moved forward and the other angle wire 71a ... 71h is moved backward, the first to the fourth bending part 68a ... 68d is rotated relative to the second to the fifth bending part 68b ... 68e, respectively.

A stopper member 78 has a circularly cylindrical shape whose distal end is substantially blocked, and the proximal end side portion of the stopper member 78 is fitted into and fixed to the distal end side portion of the first bending part 68a. The stopper member 78 is hard and insulating, and made of ceramic, for example. A substantially circularly cylindrical coupling member 82 made of metal is housed within the proximal end side portion of the stopper member 78, and the distal end side portion of the coupling member 82 having a thick diameter is fitted into and fixed to the inner peripheral surface of the stopper member 78. The distal end portion of the resin tube 84 is inserted between the stopper member 78 and the proximal end side portion of the coupling member 82 having a thin diameter, and the resin tube 84 is fitted onto and fixed to the proximal end side of the coupling member 82 having the thin diameter. The resin tube 84 is insulating and flexible, and made of fluororesin, for example. The resin tube 84 is inserted through the bending portion 66 and the insertion tube portion 76 and extended to the proximal end side.

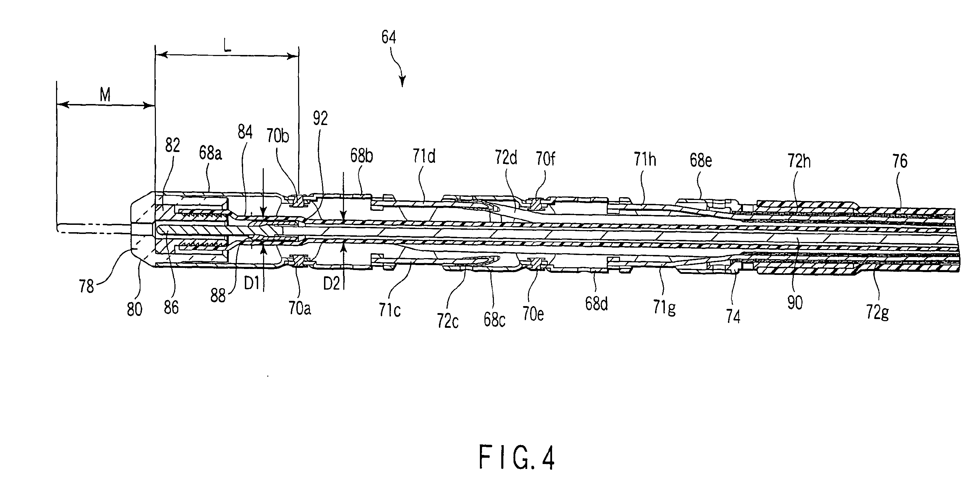

The hard and rod-shaped high-frequency electrode 86 is inserted through the central opening of the distal end wall of the stopper member 78, the inner bore of the coupling member 82 and the inner bore of the distal end portion of the resin tube 84 so as to be moved forward and backward. The distal end portion of the operation wire 90 is coupled to the proximal end portion of the high-frequency electrode 86, and a hard and substantially circularly cylindrical stopper receiving portion 88 is fitted onto and fixed to the coupling part of the high-frequency electrode 86 and the operation wire 90. The high-frequency electrode 86 and the stopper receiving portion 88 forms a treatment member, and the stopper member 78, the coupling member 82 and the resin tube 84 forms a guide member through which the high-frequency electrode 86 and the stopper receiving portion 88 is configured to be inserted so as to be moved forward and backward. The operation wire 90 is inserted through the resin tube 84 and extended to the proximal end side. Referring to FIG. 4, the inner diameter of the resin tube 84 is reduced from D1 to D2 along the centrally axial direction in the position of the first pair of coupling portions 70a and 70b arranged on the proximal end side in the first bending part 68a, and the stopper portion 92 is formed in the resin tube 84. That is, when the operation wire 90 is moved backward and then the high-frequency electrode 86 and the stopper receiving portion 88 is moved backward, the stopper receiving portion 88 is brought into contact with the stopper portion 92 of the resin tube 84 and the high-frequency electrode 86 and the stopper receiving portion 88 is positioned on a housing position. The high-frequency electrode 86 and the stopper receiving portion 88 are entirely housed within the first bending part 68a in the state where they are positioned on the housing position by the stopper portion 92. In other word, referring to FIG. 4, the total length L of the first bending part 68a along the centrally axial direction is larger than the total length of the high-frequency electrode 86 and the stopper receiving portion 88 along the centrally axial direction.

As is mentioned above, the high-frequency electrode 86 is configured to be inserted through the stopper member 78, the coupling member 82 and the resin tube 84 so as to be moved forward and backward, and the high-frequency electrode 86 is configured to be projected from and retracted into the central opening of the stopper member 78 in the centrally axial direction. Here, referring to FIG. 4, the stopper member 78 and the coupling member 82 forms a part M in which the distal end portion of the high-frequency electrode 86 is configured to be moved forward and backward, and both the stopper member 78 and the coupling member 82 are hard.

Moreover, a high-frequency current is adapted to flow through the high-frequency electrode 86 and the operation wire 90, and further, the stopper receiving portion 88 and the coupling member 82. Here, the high-frequency electrode 86, the operation wire 90, the stopper receiving portion 88 and the coupling member 82 are insulated from the first to the fifth bending part 68a ... 68e, the rivets, the first to the fourth pair of angle wires 71a ... 71h and the first to the fourth pair of coil sheaths 72a ... 72h which are made of metal with the stopper member 78 and the resin tube 84 which are insulating.

Furthermore, in order to improve an insulating performance of the bending portion 66, the rivets of the first to the fifth bending part 68a ... 68e made of metal is covered with a thin rubber tube configured not to obstruct the bending actuation of the bending portion 66. The same is true of all embodiments below.

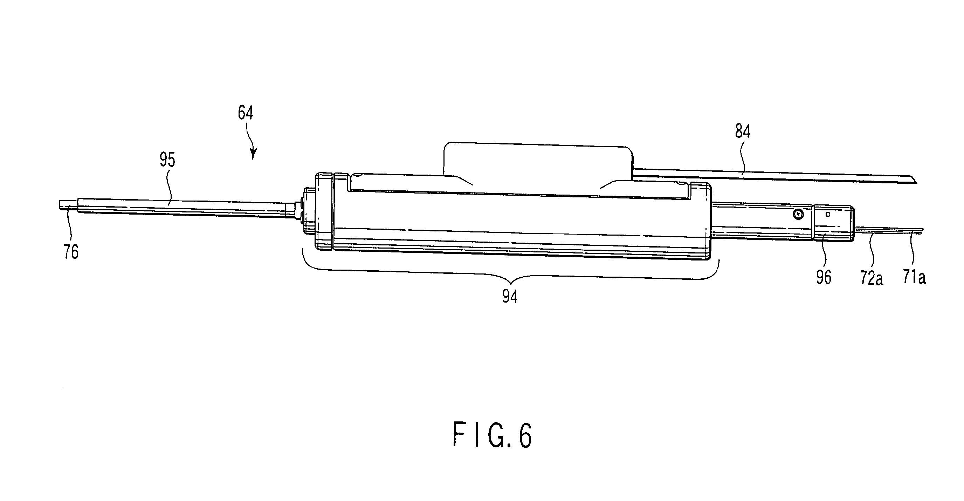

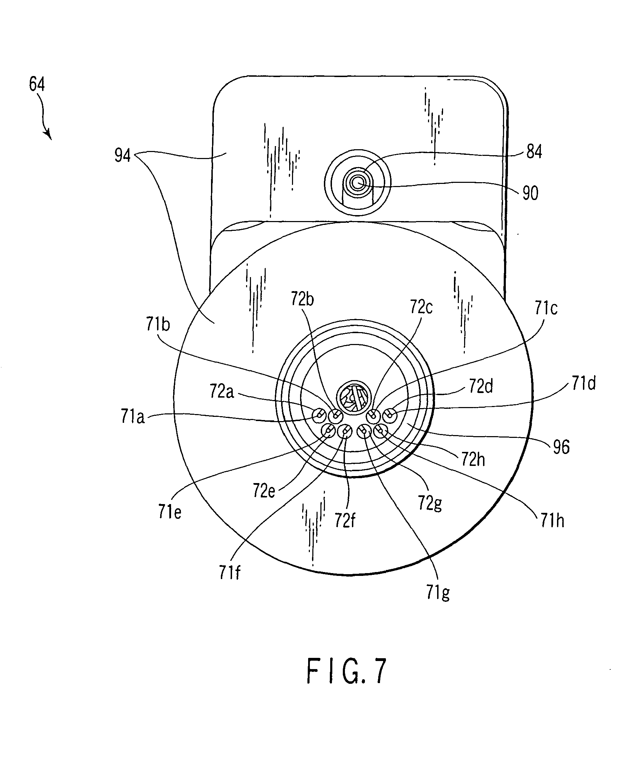

Referring to FIGS. 6 and 7, a structure of the proximal end side portion of the active high-frequency accessory 64 will be explained in detail.

The proximal end portion of the insertion tube portion 76 of the active high-frequency accessory 64 is coupled to an operation unit portion 94. In order to prevent a bending of the insertion tube portion 76 relative to the operation unit portion 94, a flexible bending preventing tube 95 is provided onto the outside of the proximal end portion of the insertion tube portion 76, and coupled to the distal end portion of the operation unit portion 94. The first to the fourth pair of angle wires 71a ... 71h and the first to the fourth pair of coil sheaths 72a ... 72h put out from the insertion tube portion 76 of the active high-frequency accessory 64 are put out from the operation unit portion 94 through a separating member 96 without tangling in each other. On the other hand, the operation wire 90 and the resin tube 84 put out from the insertion tube portion 76 of the active high-frequency accessory 64 is separated from the first to the fourth pair of angle wires 71a ... 71h and the first to the fourth pair of coil sheaths 72a ... 72h, and put out from the operation unit portion 94.

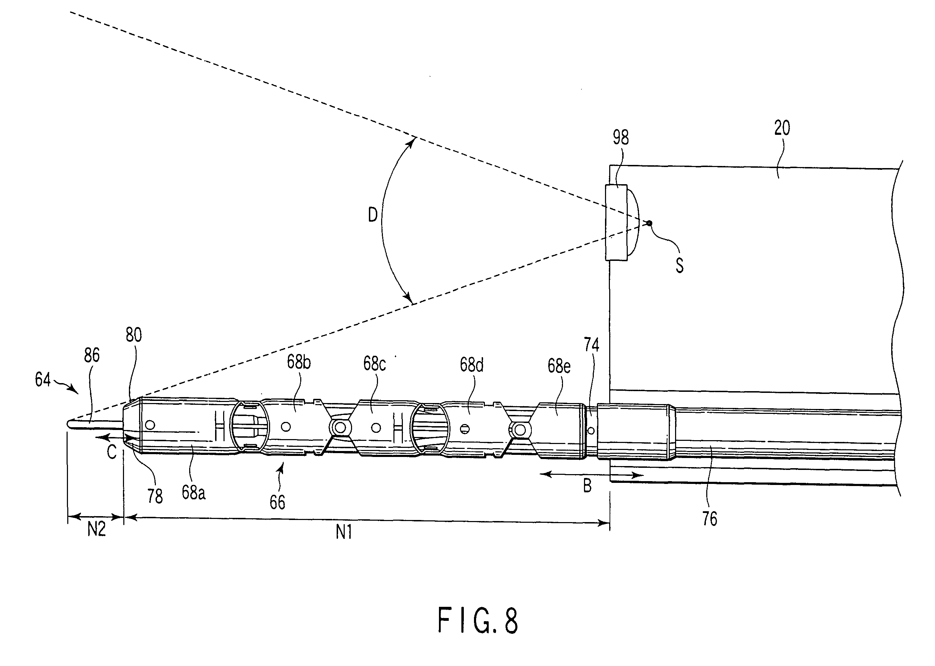

Referring to FIG. 8, a shape of the distal end portion of the active high-frequency accessory 64 will be explained.

Here, a state is referred to as a state where the active high-frequency accessory 64 is projected at a maximum, where the bending portion 66 of the active high-frequency accessory 64 is projected at a maximum N1 from the accessory channel of the active endoscope 20 without being actuated to be bent as is shown by the arrow B in FIG. 8, and further, the high-frequency electrode 86 is projected at a maximum N2 from the stopper member 78 as is shown by the arrow C in FIG. 8. Then, the shape of the distal end portion of the active high-frequency accessory 64 is configured not to block an optical axis passing through a focal point S of an objective lens 98 of the observation optical system of the active endoscope 20 and the distal end of the high-frequency electrode 86 regarding a field of view of the active endoscope 20 as is shown by the arrow D in FIG. 8 even when the active high-frequency accessory 64 is projected at the maximum. In the embodiment, a tapering portion 80 tapering toward the distal end side is formed on the outer peripheral surface of the distal end side portion of the stopper member 78 in order not to block the optical axis.

Next, a method for using the active high-frequency accessory 64 according to the embodiment will be explained.

When a living tissue is incised using the active high-frequency accessory 64 according to the embodiment, the active high-frequency accessory 64 is projected from the distal end portion of the active endoscope 20.

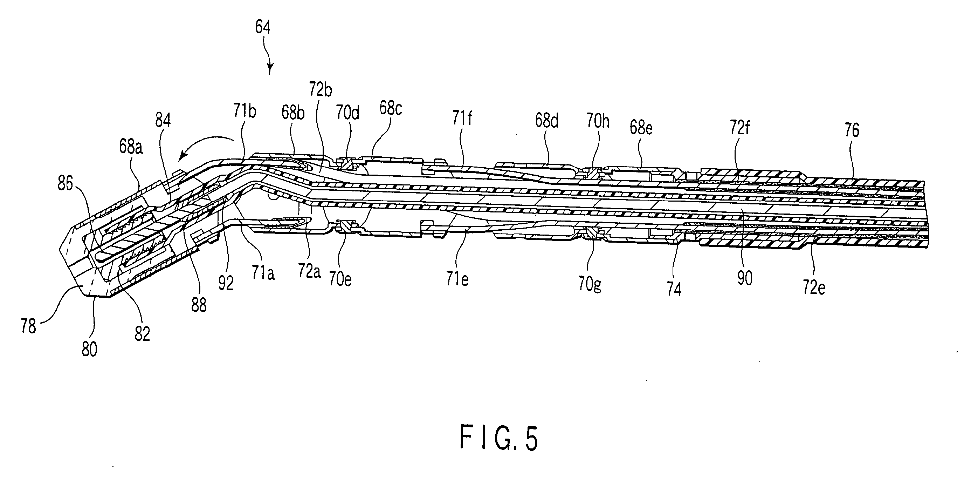

When the high-frequency electrode 86 is not used and the bending portion 66 is tried to be actuated to be bent, the slider 48 of the operation portion 46 of the active high-frequency accessory 64 is operated to be moved backward to move the high-frequency electrode 86 and the stopper receiving portion 88 backward through the operation wire 90, and then, it is judged that the stopper receiving portion 88 is brought into contact with the stopper portion 92 of the resin tube 84 in a point of time when the operation resistance is increased, and the operation to move the slider 48 backward is stopped. As a result, the high-frequency electrode 86 and the stopper receiving portion 88 are positioned on the housing position by the stopper portion 92, and the high-frequency electrode 86 and the stopper receiving portion 88 are entirely housed within the first bending part 68a. In this state, the master arm 44 is operated to actuate the bending portion 66 to be bent. In this time, as is shown in FIG. 5, the high-frequency electrode 86 and the stopper receiving portion 88 which are hard are entirely housed within the first bending part 68a, and it is prevented that a bending actuation of the bending portion 66 is obstructed due to interference between the high-frequency electrode 86 and the stopper receiving portion 88, and the bending part 68a ... 68e.

When the active high-frequency accessory 64 is used, the slider 48 of the operation portion 46 is operated to be moved forward, the high-frequency electrode 86 and the stopper receiving portion 88 are moved forward through the operation wire 90, and the high-frequency electrode 86 is projected from the stopper member 78. In this time, the distal end of the high-frequency electrode 86 is moved forward in the stopper member 78 and the coupling member 82 which are hard, and therefore, it is prevented that the stopper member 78 and the coupling member 82 are damaged by the high-frequency electrode 86.

Then, the footswitch is operated to actuate the high-frequency power source 56, and a high-frequency current is supplied from the high-frequency power source 56 through the operation wire 90 to the high-frequency electrode 86. In this time, a high-frequency current flows through the operation wire 90, the high-frequency electrode 86, the stopper receiving portion 88 and the coupling member 82, but they are insulated with the stopper member 78 and the resin tube 84, and therefore, a leakage current is prevented from being generated. Then, a living tissue is incised with the high-frequency electrode 86.

Such actuation of the distal end side portion of the active high-frequency accessory 64 is performed under observation with the endoscope. Here, even when the active high-frequency accessory 64 is projected at the maximum, the distal end of the high-frequency electrode 86 is visually recognized due to a function of the tapering portion 80 of the distal end portion of the active high-frequency accessory 64.

The active high-frequency accessory 64 according to the embodiment exhibits following effects.

In the active high-frequency accessory 64 according to the embodiment, the high-frequency electrode 86 and the stopper receiving portion 88 are positioned on the housing position by the stopper portion 92, and the whole of the high-frequency electrode 86 and the stopper receiving portion 88 is housed within the first bending part 68a. Therefore, it is surely prevented that a bending actuation of the bending portion 66 is obstructed by interference between the high-frequency electrode 86 and the stopper receiving portion 88, and the bending part 68a ... 68e.

Moreover, the distal end portion of the hard high-frequency electrode 86 is moved forward and backward in the stopper member 78 and the coupling member 82, but the stopper member 78 and the coupling member 82 are hard, and therefore, it is prevented that the stopper member 78 and the coupling member 82 is damaged by the high-frequency electrode 86.

Furthermore, even when the active high-frequency accessory 64 is projected at a maximum from the distal end portion of the active endoscope 20, the distal end of the high-frequency electrode 86 can be visually recognized, and therefore, it is possible to secure sufficient visibility and surely perform an incision. In particular, in the active high-frequency accessory 64, the bending portion 66 is relatively large and it is necessary to project the whole of such bending portion 66 from the active endoscope 20, and therefore, the amount of projection is larger and it is difficult to visually recognize the distal end of the high-frequency electrode 86. Therefore, the above effect is remarkably exhibited in the active high-frequency accessory 64.

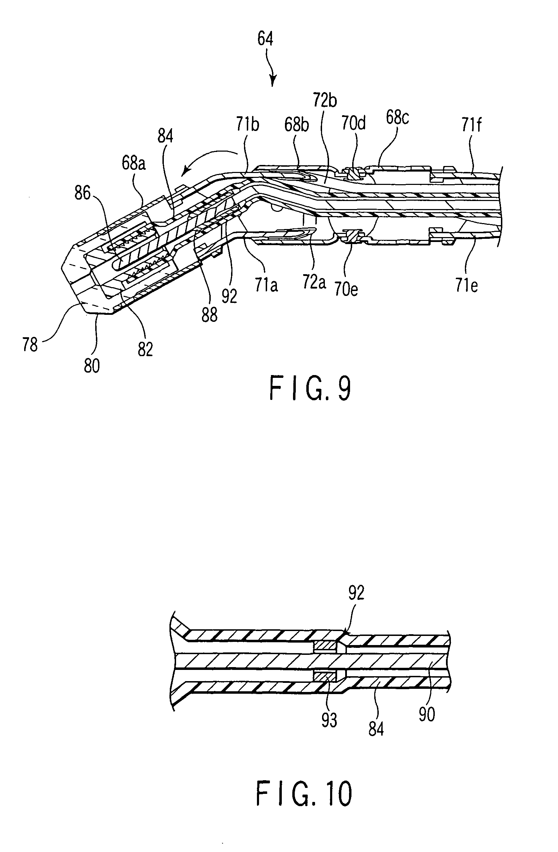

FIG. 9 shows a modified example of the first embodiment of the present invention.

In the modified example, a stopper portion 92 is arranged adjacent to the distal end side of the distal end surface of a circularly cylindrical portion of a second bending part 68b. A high-frequency electrode 86 and a stopper receiving portion 88 are slightly projected toward the proximal end side from a first bending part 68a and arranged within tongue portions of the second bending part 68b, but is not extended to the inside of the circularly cylindrical portion of the second bending part 68b in a state where they are positioned on a housing position by the stopper portion 92. In even such structure of the modified example, interference between the high-frequency electrode 86 and the stopper receiving portion 88, and the second bending part 68b is prevented.

FIG. 10 shows a second embodiment of the present invention.

In the embodiment, a hard and circularly cylindrical reinforcing member 93 is inserted into a resin tube 84, and arranged on the distal end side of a diameter decreasing portion of the resin tube 84 so as to be substantially coaxially with the resin tube 84. An operation wire 90 is inserted through the reinforcing member 93 so as to be moved forward and backward, and the diameter decreasing portion of the resin tube 84 and the reinforcing member 93 forms a stopper portion 92. A hard metal pipe and so on is used as the reinforcing member 93. In a case where the only diameter decreasing portion of the resin tube 84 forms the stopper portion 92, when a high-frequency electrode 86 is moved backward by the operation wire 90, a hard stopper receiving portion 88 may get over the stopper portion 92 and fit into the resin tube 84, and there is a possibility that a projection failure is brought about when the high-frequency electrode 86 is projected. In the embodiment, the hard reinforcing member 93 is configured to receive the stopper receiving portion 88, and therefore, there is no possibility that the stopper receiving portion 88 gets over the stopper portion 92 and is fitted into the resin tube 84, and the above projection failure is prevented.

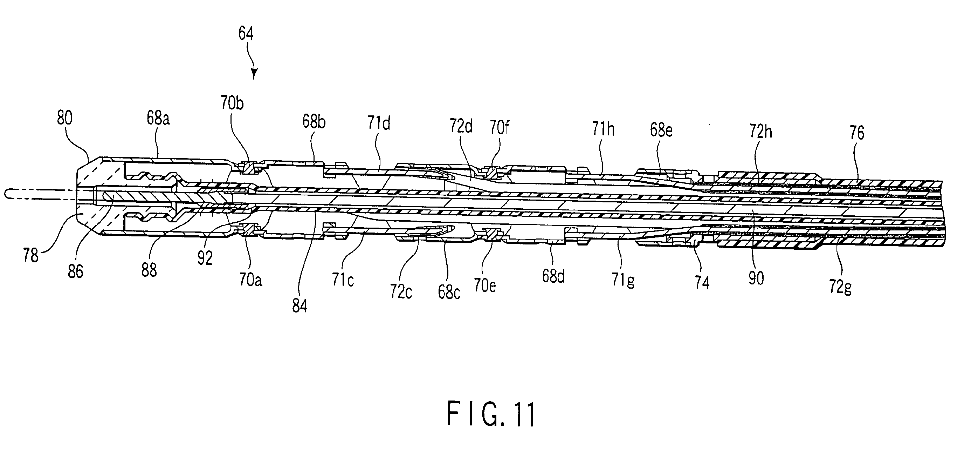

FIG. 11 shows a third embodiment of the present invention.

A stopper member 78 according to the embodiment has a shape wherein a stopper member 78 and a coupling member 82 according to the first embodiment are integrated into one body, and also, is hard and insulating, and made of ceramic, for example, as is similar to the first embodiment. In the embodiment, in comparison with the first embodiment, the number of parts is reduced, assembling performance is improved, and it is possible to inexpensively provide an active high-frequency accessory 64.

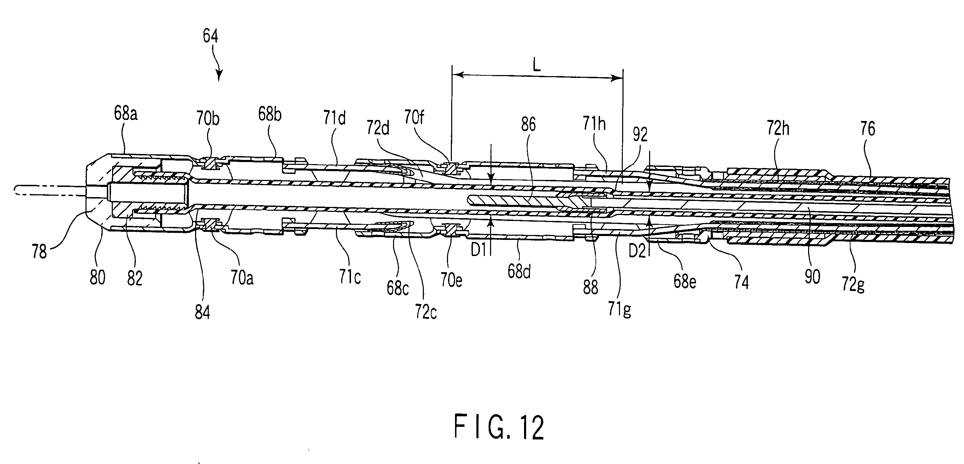

FIG. 12 shows a fourth embodiment of the present invention.

A stopper portion 92 according to the embodiment is arranged on the position of a fourth pair of coupling portions 70f and 70g (referring to FIG. 5) arranged on the proximal end side in a fourth bending part 68d with respect to the centrally axial direction. A high-frequency electrode 86 and a stopper receiving portion 88 are entirely housed within the fourth bending part 68d in a state where they are positioned by the stopper portion 92. In other word, referring to FIG. 12, the total length L of the fourth bending part 68d along the centrally axial direction is larger than the total length of the high-frequency electrode 86 and the stopper receiving portion 88 along the centrally axial direction.

A method for using an active high-frequency accessory 64 according to the embodiment is similar to the method for using the active high-frequency accessory 64 according to the first embodiment. When a slider 48 of an operation portion 46 of the active high-frequency accessory 64 is operated to be moved backward, the high-frequency electrode 86 and the stopper receiving portion 88 are moved backward from a first bending part 68a to the fourth bending part 68d by an operation wire 90, the stopper receiving portion 88 is brought into contact with the stopper portion 92, and the high-frequency electrode 86 and the stopper receiving portion 88 are positioned and entirely housed within the fourth bending part 68d.

In the active high-frequency accessory 64 according to the embodiment, the high-frequency electrode 86 and the stopper receiving portion 88 are housed within the fourth bending part 68d arranged on the proximal end side out of the first to the fifth bending part 68a ... 68e. Therefore, it is possible to make the first to the third bending part 68a, 68b, 68c arranged on the distal end side smaller, make a turning radius of the bending portion shorter, and perform easily and rapidly an incision even within a small cavity in the body.

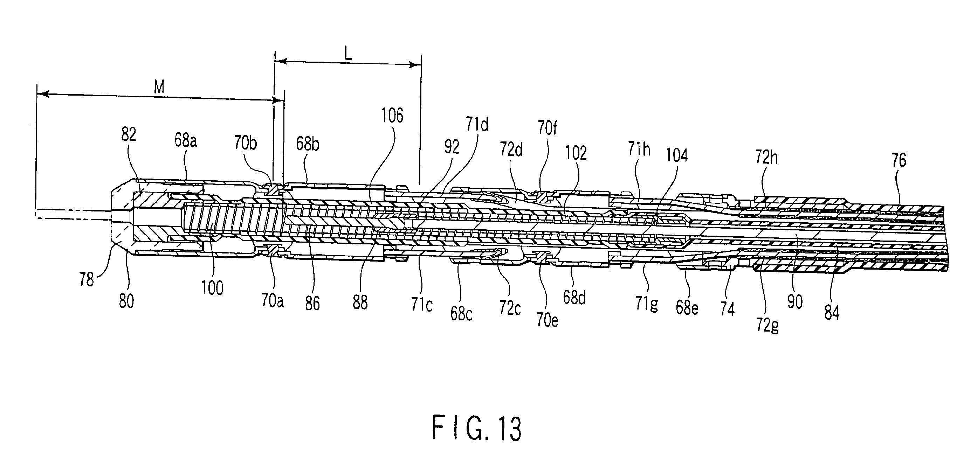

FIG. 13 shows a fifth embodiment of the present invention.

In the embodiment, the distal end portion of a thick diameter coil tube 100 is fitted into and fixed to the proximal end side portion of a coupling member 82. The distal end portion of a thin diameter coil tube 102 is fitted into and fixed to the proximal end portion of the thick diameter coil tube 100. These thick diameter coil tube 100 and thin diameter coil tube 102 are made of metal, and hard itself but sufficiently flexible on the whole. The distal end of the thin diameter coil tube 102 is arranged on the position of a second pair of coupling portions 70c and 70d (referring to FIG. 5) arranged on the proximal end side in a second bending part 68b with respect to the centrally axial direction. The distal end surface of the thin diameter coil tube 102 forms a stopper portion 92. The proximal end of the thin diameter coil tube 102 is arranged on the position of a fourth pair of coupling portions 70g and 70h (referring to FIG. 5) arranged on the proximal end side in a fourth bending part 68d with respect to the centrally axial direction. The distal end side portion of a coupling tube 104 is fitted onto and fixed to the proximal end portion of the thin diameter coil tube 102. The distal end portion of a resin tube 84 is fitted onto and fixed to the proximal end portion of the thin diameter coil tube 102 and the coupling tube 104, and the resin tube 84 is inserted through a fifth bending part 68e and an insertion tube portion 76 and extended to the proximal end side. The resin tube 84 is insulating and flexible, and made of fluororesin, for example, as is similar to the first embodiment. Furthermore, a rubber tube 106 is fit onto and fixed to the proximal end side portion of the coupling member 82, the thick diameter coil tube 100, the thin diameter coil tube 102 and the distal end portion of the resin tube 84. The rubber tube 106 is insulating and soft.

Here, a high-frequency electrode 86 is configured to be inserted through the central opening of the distal end wall of the stopper member 78, the inner bore of the coupling member 82 and the inner bore of the thick diameter coil tube 100 so as to be moved forward and backward. That is, the stopper member 78, the coupling member 82 and the thick diameter coil tube 100 forms a guide member through which the high-frequency electrode 86 as a treatment member is inserted so as to be moved forward and backward. The high-frequency electrode 86, an operation wire 90 and a stopper receiving portion 88 are assembled similarly to the first embodiment, and the operation wire 90 is inserted through the thick diameter coil tube 100, the thin diameter coil tube 102, the coupling tube 104, and the resin tube 84 in order. When the operation wire 90 is moved backward to move the high-frequency electrode 86 and the stopper receiving portion 88 backward, the stopper receiving portion 88 is brought into contact with the stopper portion 92 formed by the distal end surface of the thin diameter coil tube 102, and the high-frequency electrode 86 and the stopper receiving portion 88 are positioned. The high-frequency electrode 86 and the stopper receiving portion 88 are entirely housed within the second bending part 68b in a state where they are positioned by the stopper portion 92. In other word, the total length L of the second bending part 68b along the centrally axial direction is larger than the total length of the high-frequency electrode 86 and the stopper receiving portion 88 along the centrally axial direction.

Moreover, the stopper member 78, the coupling member 82 and the thick diameter coil tube 100 forms a part M in which the distal end portion of the high-frequency electrode 86 is adapted to be moved forward and backward, and these stopper member 78, coupling member 82 and thick diameter coil tube 100 are hard.

Furthermore, a high-frequency current is adapted to flow through the high-frequency electrode 86 and the operation wire 90, and further, the stopper receiving portion 88, the coupling member 82, the thick diameter coil tube 100, the thin diameter coil tube 102, and the coupling tube 104. Here, the high-frequency electrode 86, the operation wire 90, the stopper receiving portion 88, the coupling member 82, the thick diameter coil tube 100, the thin diameter coil tube 102 and the coupling tube 104 is insulated from a first to the fifth bending part 68a ... 68e, rivets, a first to a fourth pair of angle wires 71a ... 71h and a first to a fourth pair of coil sheaths 72a ... 72h which are made of metal with the stopper member 78, the rubber tube 106 and the resin tube 84 which are insulating.

A method for using an active high-frequency accessory 64 according to the embodiment is similar to the method for using the active high-frequency accessory 64 according to the first embodiment. When a slider 48 of an operation portion 46 of the active high-frequency accessory 64 is operated to be moved backward, the high-frequency electrode 86 and the stopper receiving portion 88 are moved backward from the first bending part 68a to the second bending part 68b by the operation wire 90, the stopper receiving portion 88 is brought into contact with the stopper portion 92, and the high-frequency electrode 86 and the stopper receiving portion 88 are positioned and entirely housed within the second bending part 68b.

The active high-frequency accessory 64 according to the embodiment exhibits a following effect.

In the embodiment, in the bending portion 66, the thick diameter coil tube 100 and thin diameter coil tube 102, and the rubber tube 106 fitted onto and fixed to them is used as what the high-frequency electrode 86, the stopper receiving portion 88, and the operation wire 90 are inserted through so as to be movable forward and backward. The thick diameter coil tube 100 and the thin diameter coil tube 102 which are sufficient flexible hardly obstructs a bending actuation of the bending portion 66 and is hardly buckled due to the bending actuation, and the inner diameter thereof is secured, and therefore, a forward and backward movement of the high-frequency electrode 86, the stopper receiving portion 88 and the operation wire 90 is hardly obstructed. Moreover, the thick diameter coil tube 100 is hard itself, and therefore, prevented from being damaged due to a forward and backward movement of the high-frequency electrode 86. Furthermore, the rubber tube 106 is soft, and therefore, hardly obstructs a bending actuation of the bending portion 66, and also, the rubber tube 106 is insulating, and therefore, prevents a leakage current. In this way, a combination of the thick diameter coil tube 100, the thin diameter coil tube 102 and the rubber tube 106 realizes an excellent bending performance, forward and backward movability, and insulating ability.

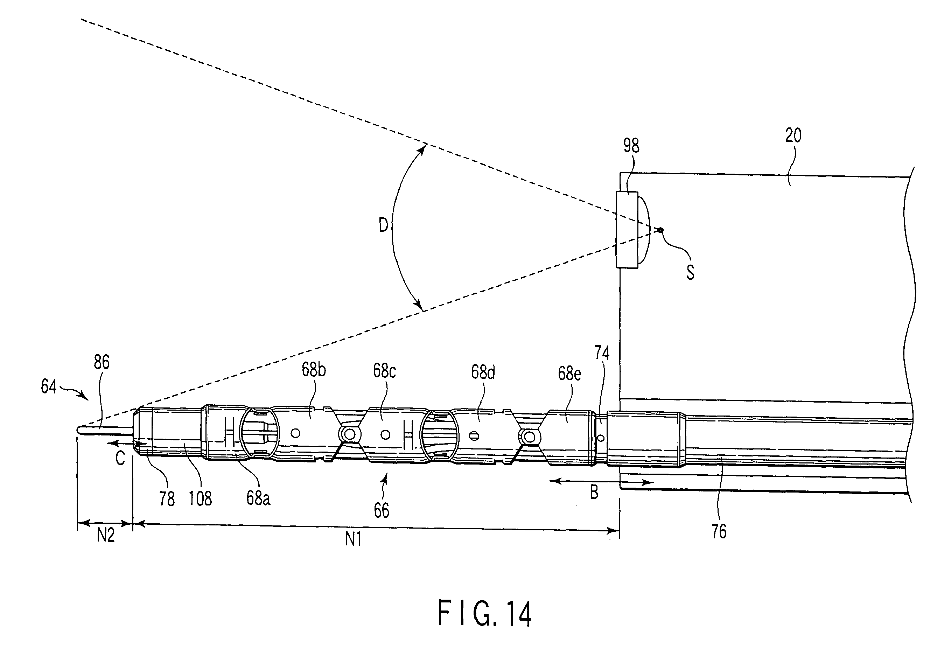

FIG. 14 shows a sixth embodiment of the present invention.

In an active high-frequency accessory 64 according to the embodiment, a shape of the distal end portion configured to secure visibility is different from that of the first embodiment. That is, a distal end side portion 108 of a first bending part 68a and a stopper member 78 has a thinner diameter than that of a proximal end side portion of the first bending part 68a so as not to block an optical axis. In the embodiment, the shape of the distal end side portion 108 of the first bending part 68a and the stopper member 78 is similar to a shape of a distal end portion of a conventional high-frequency accessory, and therefore, it is possible to perform an incision at a similar operation feeling to that of the conventional high-frequency accessory.

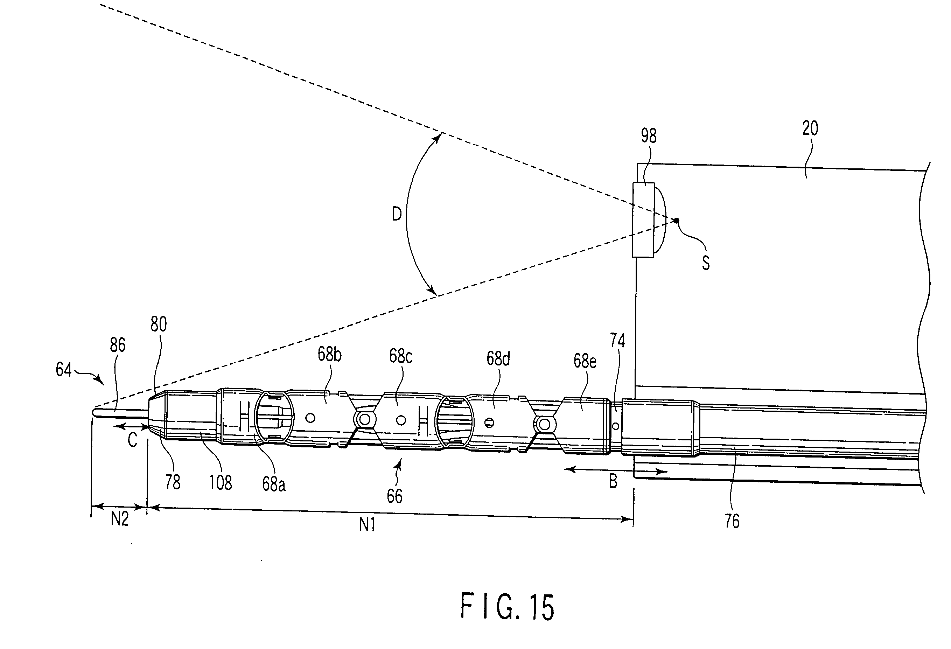

FIG. 15 shows a seventh embodiment according to the present invention.

In an active high-frequency accessory 64 according to the embodiment, a shape of the distal end portion configured to secure visibility is different from that of the first embodiment, as is similar to the sixth embodiment. That is, a distal end side portion 108 of a first bending part 68a and a stopper member 78 has a thinner diameter than that of a proximal end side portion of the first bending part 68a, and further, a tapering portion 80 tapering toward the distal end side is formed on the outer peripheral surface of the distal end side portion of the stopper member 78. In the embodiment, the shape of the distal end portion more hardly blocks an optical axis than that of the first and the sixth embodiment. Therefore, the distal end of the high-frequency electrode 86 can be surely visually recognized, and therefore, it is possible to further secure sufficient visibility and securely perform an incision.

In the above mentioned embodiment, an active high-frequency accessory is explained as an example, the present invention may be applied to various accessories configured to house a relatively hard treatment member within a bending portion. For example, it may be applied to an accessory for a local injection using a hard injection needle as a treatment member.

## Claims

1. An accessory comprising:
bending parts forming a bending portion wherein each bending part includes a cylindrical portion and a coupling end portion, the cylindrical portions are arranged side by side in an axial direction, and both the coupling end portions adjacent to each other are coupled to each other so as to be rotatable relative to each other;
a cylindrical guide member extending within the bending portion;
a treatment member configured to be inserted through the guide member so as to be movable forward and backward and placed in a housing position where the whole treatment member is housed within at least a given bending part of the bending parts out of the given bending part and the coupling end portion provided in the bending part adjacent to the given bending part and arranged on a side close to the given bending part; and
a stopper portion provided on the guide member and configured to be brought into contact with the treatment member to position the treatment member on the housing position.

2. The accessory according to claim 1,
wherein the whole treatment member is configured to be housed within the given bending part when the treatment member is placed on the housing position.

3. The accessory according to claim 1,
wherein the treatment member is configured to be housed within the given bending part other than the bending part arranged on a distal end out of the bending parts.

4. The accessory according to claim 1,
wherein a part of the guide member, in which the treatment member is adapted to be moved forward and backward, is hard.

5. The accessory according to claim 1,
wherein the accessory is configured to be inserted through an accessory channel of an endoscope and projected from a distal end portion of the endoscope, and the distal end portion of the accessory has a shape configured not block an optical axis passing through a focal point of an observation optical system of the endoscope and a distal end of the accessory when the accessory is projected at a maximum from the distal end portion of the endoscope.
